# EUROPÄISCHE PATENTSCHRIFT

(11) **EP 0 809 608 B1**
(45) Veröffentlichungstag und Bekanntmachung des Hinweises auf die Patenterteilung: **07.01.1999**
(21) Anmeldenummer: 96904016.1
(22) Anmeldetag: 08.02.1996
(51) Int. Cl.: C02F 1/70, C02F 3/12, C02F 3/10

(54) **KOMBINIERTES VERFAHREN ZUR CHEMISCHEN UND BIOLOGISCHEN BEHANDLUNG VON WASSER**
COMBINED CHEMICAL AND BIOLOGICAL WATER TREATMENT PROCESS
PROCEDE COMBINE DE TRAITEMENT CHIMIQUE ET BIOLOGIQUE DE L'EAU

(30) Priorität: 17.02.1995 DE 19505436
(43) Veröffentlichungstag der Anmeldung: 03.12.1997
(73) Patentinhaber: Solvay Umweltchemie GmbH, D-30173 Hannover (DE)
(72) Erfinder: BONSE, Dirk, D-31275 Lehrte (DE); SELL, Michael, D-31228 Peine (DE)
(74) Vertreter: Gosmann, Martin
(86) Internationale Anmeldenummer: EP9600518
(87) Internationale Veröffentlichungsnummer: WO9625364

(56) Entgegenhaltungen:
- EP-A- 0 204 273
- WO-A-93/17786
- DE-A- 3 923 514
- PATENT ABSTRACTS OF JAPAN vol. 017, no. 205 (C-1051), 22.April 1993 & JP,A,04 349996 (JAPAN ORGANO CO LTD), 4.Dezember 1992,
- PATENT ABSTRACTS OF JAPAN vol. 018, no. 482 (C-1247), 8.September 1994 & JP,A,06 154787 (JAPAN ORGANO CO LTD), 3.Juni 1994,
- PATENT ABSTRACTS OF JAPAN vol. 018, no. 482 (C-1247), 8.September 1994 & JP,A,06 154786 (JAPAN ORGANO CO LTD), 3.Juni 1994,

## Beschreibung

Die Erfindung betrifft die Entfernung oder Verminderung des Nitrat- und/oder Nitrit-Gehaltes in Wasser mittels eines kombinierten biologisch/chemisch-katalytisch reduktiven Verfahrens zur Wasserbehandlung. Die Erfindung betrifft weiterhin ein Verfahren zur Aktivierung bzw. Selektion von hochleistungsfähigen Nitrat- und/oder Nitrit-abbauenden Mikroorganismen unter Verwendung von Trägern oder Katalysatoren aus Nitrat- und/oder Nitritreduktionsverfahren, sowie die hierbei gewonnenen Nitrat- und/oder Nitrit-abbauenden Mikroorganismen selbst.

Anwendungsgebiete der vorliegenden Erfindung sind die Mikrobiologie und der Umweltschutz (Wasserbehandlung), insbesondere die Trinkwasserbehandlung und Wasseraufbereitung, z. B. die Brauch- und die Abwasserreinigung.

Es ist bekannt, daß zahlreiche Mikroorganismen zur Beseitigung von Verunreinigungen und von Schadstoffen eingesetzt werden. Dabei werden geeignete Kulturen in die verunreinigten Materialien eingebracht, bzw. mit ihnen vermischt. So werden Mikroorganismen-Kulturen z.B. in der Abwasserbehandlung eingesetzt, wobei man entweder die Kulturen mit dem Abwasser vermischt oder einen Rasen aus Mikroorganismen auf Trägern herstellt, über den man das Abwasser rieseln läßt. Übliche Träger für diesen Einsatzzweck sind keramische Stoffe, Glaskugeln, zerkleinerte Lava Schlacke oder Aktivkohle.

Bei der Trinkwasseraufbereitung stellt die Belastung des Grund- und Oberflächenwassers mit Nitrat und/oder Nitrit ein zunehmendes Problem dar. So sind die in Grund- und Oberflächenwasser in der Bundesrepublik Deutschland aufgefundenen Nitrit- und/oder Nitratgehalte in den letzten Jahren stark angestiegen. Sie schwanken je nach Standort erheblich und können teilweise die tolerierbaren Höchstgrenzen überschreiten. Für Trinkwasser wird ein zulässiger Grenzwert von 50 mg Nitrat/l vorgeschrieben; für Nitrit ein Grenzwert von 0,1 mg/l. Insbesondere die steigenden Nitratgehalte in vielen Grund- und Oberflächenwassern machen in zunehmendem Maße Verfahren zur Nitrat- und Nitritentfernung im Rahmen der Brauch- und Trinkwasseraufbereitung nötig.

Zur Nitrat- und Nitritentfernung aus Trinkwasser stehen neben physikalisch-chemischen Maßnahmen auch biologische Verfahren zur Verfügung. In der biologischen Denitrifikation werden Bakterien eingesetzt, welche in Abwesenheit von Sauerstoff Nitrat und Nitrit als terminalen Wasserstoffakzeptor verwenden können. Nachteil dieser Methode ist die Gefahr der Kontamination des Trinkwassers mit Bakterien, so daß eine Nachreinigung des so behandelten Wassers nötig ist. Ein weiteres Problem bei biologischen Verfahren besteht darin, daß pathogene Keime bei der Trinkwasseraufbereitung unbedingt zu vermeiden sind und daß die Bedingungen daher so eingestellt sein müssen, daß nur gesundheitlich unbedenkliche, für den Nitrat- und/oder Nitritabbau erforderlichen Mikroorganismen akzeptable Lebensbedingungen vorfinden. Die Mikroorganismen sollen dabei hohe Nitrat- und/oder Nitritabbauleistungen aufweisen.

Aufgabe der vorliegenden Erfindung ist es, ein neues wirtschaftlich arbeitendes und sicheres chemisch-biologisches Verfahren zur Entfernung von Nitrat und/oder Nitrit aus Wasser bereitzustellen. Insbesondere ist es die Aufgabe der Erfindung, ein kombiniertes biologisch/chemisch-katalytisch reduktives Verfahren zur Entfernung von Nitrat- und/oder Nitrit aus Nitrat- und/oder Nitrit-belastetem Wasser zu entwikkeln, welches unter den bei der Wasserbehandlung üblichen Bedingungen durchführbar ist, und bei dem der Nitrat- und/oder Nitrit-Gehalt unter Bildung von gasförmigen Produkten, insbesondere Stickstoff, entfernt wird, ohne daß dabei das Wasser mit Keimen belastet wird.

Eine weitere Aufgabe der vorliegenden Erfindung ist es, ein neues Verfahren zur Aktivierung und/oder zur Selektion von hochleistungsfähigen Nitrat- und/oder Nitrit-abbauenden Mikroorganismen, sowie die hierdurch erhältlichen Mikroorganismen, zur Verfügung zu stellen.

Insbesondere ist es Aufgabe der Erfindung, solche Mikroorganismen zur Entfernung von Nitrit und/oder Nitrat aus Nitrit- und/oder nitratbelasteten Wassern zu aktivieren oder zu selektieren, welche unter den bei der Wasseraufbereitung üblichen Bedingungen - gegebenenfalls auch in Kombination mit chemisch-katalytischen Reduktionsverfahren - einsetzbar sind.

Diese Aufgabe wird gelöst durch das in den Ansprüchen angegebene Verfahren und die nachfolgend hierzu gegebenen Erläuterungen. Die Erfindung betrifft daher ein Verfahren zur Entfernung oder Verminderung des Nitrat- und/oder Nitrit-Gehaltes in Nitrat- und/oder Nitrit-belastetem Wasser unter selektiver Stickstoffbildung, welches sich dadurch auszeichnet, daß man das Nitrat- und/oder Nitrit-belastete Wasser unter Zudosierung von Wasserstoffgas und von wasserlöslichem anorganischem Desinfektionsmittel unter anoxischen Bedingungen mit einer auf einem anorganischen oder organischen Träger angesiedelten, hochleistungsfähigen Nitrat- und/oder Nitrit-abbauenden mikrobiologischen Flora kontaktiert, wobei diese mikrobiologische Flora aus natürlicherweise im zu behandelnden Wasser vorhandenen Mikroorganismen vor und/oder während des Verfahrens unter dem Selektionsdruck des zudosierten wasserlöslichen anorganischen Desinfektionsmittels und den anoxischen Bedingungen auf dem Träger selbst anhaftend kolonisiert und dabei an die Verfahrensbedingungen zur Entfernung oder Verminderung des Nitrat-und/oder Nitrit-Gehaltes aus dem Wasser adaptiert wird.

Zweckmäßig wird das vorstehende Verfahren so ausgeführt, daß man das Nitrat- und/oder Nitrit-belastete Wasser unter Zudosierung von Wasserstoffgas und eines anorganischen wasserlöslichen Desinfektionsmittels
a) zur Herstellung von anoxischen Bedingungen zunächst über einen mit Katalysator befüllten Reaktor R1 führt, in dem im Wasser enthaltener Sauerstoff mit Wasserstoff katalytisch reduziert wird,
b) das Wasser über einen zweiten, mit einem anorganischen oder organischen Träger befüllten Reaktor R2 führt, der zur Ansiedlung einer hochleistungsfähigen Nitrat- und/-oder Nitrit-abbauenden mikrobiologischen Flora dient, wobei diese mikrobiologische Flora aus natürlicherweise im zu behandelnden Wasser vorhandenen Mikroorganismen unter dem Selektionsdruck des zudosierten wasserlöslichen anorganischen Desinfektionsmittels und den anoxischen Bedingungen auf dem Träger selbst anhaftend kolonisiert und an die Verfahrensbedingungen zur Entfernung oder Verminderung des Nitrat- und/oder Nitrit-Gehaltes aus dem Wasser adaptiert wird,
c) und man danach das Wasser, gegegebenfalls unter Zudosierung von weiterem Desinfektionsmittel und/oder Wasserstoffgas, über zumindest einen weiteren mit Katalysator zur Nitrat- und/oder Nitrit-Entfernung gefüllten Reaktor R3, führt und danach das Wasser aus dem Verfahren entläßt. Gegebenenfalls kann man noch einen oder mehrere Reaktoren nachschalten.

Im vorstehenden Verfahren wird zweckmäßigerweise als Desinfektionsmittel ein oxidierend wirkendes anorganisches Desinfektionsmittel eingesetzt, vorzugsweise Hypochlorit in der Salzform, insbesondere als Natriumsalz, oder Chlor oder Chlordioxid.

Das zudosierte Desinfektionsmittel, welches bereits ausreichend gewirkt hat, wird im Reaktor R1 ebenfalls reduziert.

Vorteilhaft wird hierbei als stark oxidierend wirkendes anorganisches Desinfektionsmittel Hypochlorit, insbesondere Natriumhypochlorit, eingesetzt.

Im erfindungsgemäßen Verfahren wird das Desinfektionsmittel, z. B. das Natriumhypochlorit, in einer Menge von 0,05 bis 0,5 mg/l Wasser (berechnet als freies Aktivchlor), zudosiert; vorzugsweise in einer Menge von 0,1 bis 0,3 mg/l; jeweils bezogen auf das zu behandelnde Wasser.

Das erfindungsgemäße Verfahren ermöglicht es, die Aktivierung und/oder Selektion der Mikroorganismen unter Bedingungen durchzuführen, wie sie bei der katalytisch-reduktiven Wasseraufbereitung zur Entfernung von Nitrat und/oder Nitrit angewendet werden. Die mikrobiologische Flora kann auf Träger in bestehenden Anlagen in einfacher Weise während der Anlaufphase aufwachsen, bis ein stationärer Zustand erreicht ist und die Anlage danach zum Nitrat- und/oder Nitrit-Abbau weiter betrieben werden. Hierbei können die durch Ansiedlung mit den aktivierten und/oder selektierten Mikroorganismen besiedelten Träger ohne Isolierung der Mikroorganismen direkt nach dem Aktivierungs- und/oder Selektionsprozeß zum Nitrat/Nitritabbau von mit Nitrat und/oder Nitrit belastetem Wasser weiterverwendet werden. Die nach dem erfindungsgemäßen Verfahren aktivierten und/oder selektierten Mikroorganismen lassen sich aber auch nach an sich üblichen mikrobiologischen Verfahren unter dem Selektionsdruck entsprechender Milieubedingungen isolieren und in bestehende Anlagen einimpfen.

Die selektierten Mikroorganismen können auf einem anorganischen Träger oder auf einem organischen Polymerträger angesiedelt werden, wobei der Träger sowohl unporös als auch porös beschaffen sein kann. Als organische Polymerträger kommen an sich übliche für die Immobilisierung von Mikroorganismen geeignete Träger in Betracht, die unter den Verfahrensbedingungen stabil sind. Bevorzugt sind aber anorganische Träger, insbesondere solche mit guter Abriebfestigkeit und Hydrolysebeständigkeit; Beispiele für anorganische Träger sind Träger auf Basis von Glas, Siliziumdioxid, Al₂O₃, TiO₂, ZrO₂ - z.B. in Form von Kügelchen - und auch die nachfolgend näher erläuterten Träger. Vorteilhaft können anorganische poröse Träger eingesetzt werden, die bereits in katalytisch-reduktiven Wasserbehandlungsverfahren Verwendung finden.

Das Verfahren der Erfindung zeichnet sich daher in einer vorteilhaften Ausführungsart dadurch aus, daß die mikrobiologische Flora auf einem anorganischen Träger angesiedelt ist, vorzugsweise auf einem anorganischen porösen Träger, welcher im wesentlichen aus Aluminiumoxid (Al₂O₃) besteht und durch folgende Eigenschaften gekennzeichnet ist:
aI) er besteht aus Aluminiumoxid der Modifikation "Gamma" und "Kappa", wobei die Modifikation "Delta" in geringer Menge enthalten sein kann, oder
aII) er besteht aus Aluminiumoxid der Modifikation "Theta" und "Kappa", wobei die Modifikation "Alpha" in geringer Menge enthalten sein kann;
b) die Verteilung der Porendurchmesser weist ein Maximum (Monomodale Porenverteilung) oder zwei Maxima (bimodale Porenverteilung) im Bereich zwischen 100 und 1500 Å (= 10 und 150 nm) auf;
c) die BET-Oberfläche liegt zwischen 20 und 200 m²/g.

Bevorzugt sind im wesentlichen aus Aluminiumoxid bestehende Träger, die entweder ein Maximum (monomodale Porenverteilung) in der Porenverteilung im Bereich von 500 bis 1500 Å (= 50 bis 150 nm) aufweisen oder zwei Maxima (bimodale Porenverteilung) in der Porenverteilung im Bereich von 100 bis 1500 Å (= 10 bis 150 nm) aufweisen.

Eine andere Ausführung des erfindungsgemäßen Verfahrens zeichnet sich dadurch aus, daß ein anorganischer Träger auf Basis von Aluminiumoxid - ggf. mit in das Aluminiumoxid eingebrachten Anteilen anorganischer Oxide aus der Gruppe Magnesiumoxid, Siliciumdioxid, Alumosilikat, Titandioxid, Manganoxid, Eisenoxid, Molybdänoxid, Vanadiumoxid und/oder Zirkoniumoxid - eingesetzt wird, wobei der Träger entweder durch Granulation oder durch Überführen eines Sols in kugelförmige Solteilchen unter Kontaktieren der Solteilchen mit einem Reaktionsgas und Auffangen der Solteilchen in einer Auffangvorrichtung, Aufarbeiten der verfestigten Solteilchen durch Altern, Waschen, Trocknen und Kalzinieren, hergestellt wird, mit der Maßgabe, daß der Träger eine spezifische BET-Oberfläche im Bereich von 5 bis 250 m²/g, vorzugsweise im Bereich 100 bis 200 m²/g aufweist. Die Herstellung von solchen Trägern wird insbesondere in der WO 94/20203 beschrieben; insbesondere in poröser Form.

Die vorstehenden Ausführungsarten der Träger können als solche im erfindungsgemäßen Verfahren zur Besiedelung mit Mikroorganismen eingesetzt werden, oder sie können auch zuvor mit einem katalytisch wirkenden Übergangsmetall dotiert und/-oder imprägniert werden, vorzugsweise mit einem oder mehreren Metallen aus der Gruppe Palladium, Rhodium und Kupfer. Als sehr vorteilhaft hat sich u.a. der Einsatz von solchen anorganischen porösen Trägern der vorstehend beschriebenen Art erwiesen, die mit Palladium und/oder Kupfer imprägniert sind.

Gemäß der Erfindung wurde somit ein kontinuierlich durchführbares biologisches, insbesondere biologisch/ chemisch-katalytisches Reduktionsverfahren gefunden, mit welchem Nitrat und/oder Nitrit aus Wasser unter Bildung von gasförmigen Reduktionsprodukten, überwiegend Stickstoff, entfernt werden können, und zwar unter Vermeidung von Keimbelastung. Dazu trägt die selektierte mikrobiologische Flora in Kombination mit insbesondere den bevorzugt eingesetzten Trägern zur stabilen, selbsthaftenden Ansiedlung in überraschender Weise bei. Im Rahmen der vorliegenden Erfindung können daher Nitrat- oder Nitrit-belastete Wasser und wäßrige Lösungen behandelt werden, wie sie ganz allgemein im Stand der Technik üblicherweise auch den chemisch-reduktiven Verfahren zur Wasseraufbereitung zugeführt werden; daher bezeichnet der Ausdruck "Wasser" in der vorliegenden Anmeldung derartige Wässer und wäßrige Lösungen. Der Nitrat- und/oder Nitrit-Gehalt, welcher mit dem erfindungsgemäßen Verfahren entfernt oder auf eine tolerierbare Menge vermindert werden kann, kann in einem weiten Bereich variieren. So lassen sich mit dem erfindungsgemäßen Verfahren Wasser mit einer Nitrat- und/oder Nitrit-Belastung zwischen 0,1 mg/l und einigen g pro 1 behandeln. Insbesondere ist das vorliegende Verfahren geeignet zur Entfernung des Nitrat- und/oder Nitrit-Gehaltes aus Wasser mit einer geringeren Nitrat- und/oder Nitrit-Belastung, beispielsweise im Bereich von etwa 10 bis 300 mg/l Nitrat und/-oder von etwa 0,1 bis etwa 20 mg/l Nitrit. Insbesondere wird das erfindungsgemäße Verfahren zur Entfernung von Nitrat- und/oder Nitrit aus Wasser eingesetzt, welches in seinem Reinheitsgrad einem Wasser entspricht, welches eine natürliche Filtration durchlaufen hat. Derartiges Wasser kann wasserlösliche Substanzen, z.B. anorganische Salze, in Größenordnungen, wie sie im Grundwasser anzutreffen sind, also z.B. bis zu einigen g pro 1, enthalten. Beispiele von mit dem erfindungsgemäßen Verfahren zu behandelnden Nitrat- oder Nitrit-belasteten Wassern sind z.B. Grundwasser, Brunnenwasser, Quellwasser oder Uferfiltrate oder bereits entsprechend vorgereinigte sonstige Abwässer, z.B. industrielle Abwässer, beispielsweise aus Rauchgaswäschen, aber auch Getränke wie Mineralwässer, Limonaden und Fruchtsäfte. Das Verfahren eignet sich auch beispielsweise zur Anwendung im Rahmen der Trinkwasseraufbereitung sowie der Aufbereitung von Brauchwasser für die Lebensmittel- oder Getränkeindustrie sowie für sonstige Zwecke, wo ein Nitrat- und/oder Nitrit-armes oder -freies und sauerstoffreies oder zumindest sauerstoffarmes Wasser benötigt wird.

Insgesamt werden die üblichen Verfahrensparameter beim erfindungsgemäßen Verfahren so eingestellt, wie diese bereits im wesentlichen aus den bekannten chemisch-katalytischen Reduktionsverfahren zur Entfernung von Nitrat und/oder Nitrit aus Wasser bekannt sind. Im Hinblick auf die bei der chemisch-katalytischen Wasseraufbereitung einzuhaltenden Verfahrensbedingungen wird auf die Europäische Patentanmeldung EP 359 074 hingewiesen, in der die entsprechenden Parameter wie pH-Wert, Zudosierung des Wasserstoffgases, Druck, Art der chemisch-reduktiv wirkenden Katalysatoren und andere Einzelheiten der üblichen Verfahrensführung beschrieben sind. Ferner ist auf die drei internationalen Patentanmeldungen WO 93/17786, WO 93/17790, WO 94/20202 und WO 94/20203 hinzuweisen, in denen spezielle poröse anorganische Träger sowie auch mit Metallen imprägnierte poröse anorganische Träger im Rahmen der chemisch-katalytisch reduktiven Wasseraufbereitung durch Nitrat- und/oder Nitrit-Abbau beschrieben sind. Aus den genannten internationalen Patentanmeldungen lassen sich insbesondere auch die Herstellungsweisen der weiter oben beschriebenen, bevorzugten anorganischen porösen Träger entnehmen.

Wird das erfindungsgemäße Verfahren lediglich mit den in den vorstehenden Anmeldungen beschriebenen Trägern (d.h. ohne katalytisch-aktive Metallkomponente) betrieben, so kann es als rein biologisches Verfahren zur Nitrat- und/oder Nitrit-Entfernung oder -Verminderung durchgeführt werden. Im Falle von Trägern, die zusätzlich mit einer katalytisch-aktiven Metallkomponente imprägniert sind, beispielsweise mit den Metallen Palladium, Rhodium und/oder Kupfer wie vorstehend beschrieben, kann das Verfahren sowohl in einem Reaktor als auch in mehreren Reaktoren unter anoxischen Bedingungen als kombiniertes biologisch/chemisch-katalytisch reduktives Wasserbehandlungsverfahren durchgeführt werden. Bevorzugt wird allerdings eine Reaktorkaskade eingesetzt, bei welcher zunächst die anoxischen Bedingungen chemisch-katalytisch eingestellt werden (vorzugsweise mit einem der beschriebenen Katalysatoren zur Nitrat- und/oder Nitrit-Reduktion) und erst in einem zweiten Reaktor eine mikrobielle Flora kolonisiert wird. Vorzugsweise wird im Falle des kombinierten biologisch/chemisch-katalytisch reduktiven Wasserbehandlungsverfahren mindestens noch ein chemisch-katalytisch wirkender Nitrat-und/oder Nitrit-abbauender Reaktor nachgeschaltet.

Im Verlaufe des erfindungsgemäßen Verfahrens entwickelt sich im System unter den anoxischen Bedingungen eine mikrobielle Flora, die in der Lage ist, Wasserstoff als Energiequelle, Kohlendioxid als Kohlenstoffquelle und insbesondere Nitrat als Elektronenakzeptor zu nutzen. Bevorzugt wird daher eine solche mikrobielle Flora angesiedelt, welche mit hoher Effizienz vorzugsweise Nitrat in Nitrit umwandelt, wobei der biologische Umsatz von Nitrat zu Nitrit den bei bekannten, nur chemisch-katalytischen Wasserbehandlungsverfahren stattfindenden Umsatz bei weitem übersteigt. Das gebildete Nitrit kann danach, einfacher als das Nitrat, chemisch-katalytisch in an sich bekannter Weise mit den Katalysatoren, wie sie in den zuvor zitierten internationalen Patentanmeldungen oder der genannten europäischen Patentanmeldung beschrieben sind, zu Stickstoff reduziert werden. Insgesamt wird also durch die zusätzliche mikrobiologische Flora, die Effizienz der chemisch-katalytischen Verfahren beträchtlich erhöht.

Bei der sich ansiedelnden mikrobiellen Flora handelt es sich um chemolithoautotrophe Bakterien, die im Grundwasser enthalten sind und dort normalerweise ein armseliges Kümmerdasein führen. Sie wachsen in ihrem natürlichen Milieu äußerst langsam und haben für die Qualität des Wassers eine untergeordnete Bedeutung. Diese Bakterien sind fakultativ anaerob und denitrifizieren in der Regel nur unter anoxischen Verhältnissen. Typische Vertreter dieser Gruppe sind beispielsweise Pseudomonas, Aeromonas, Alcaligenes und Paracoccus.

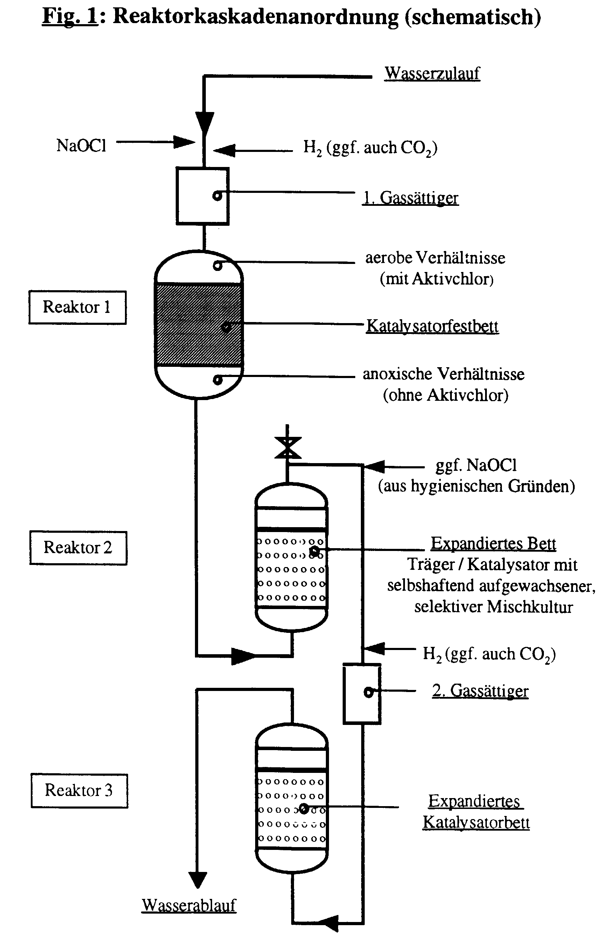

Eine beispielhafte Reaktorkaskaden-Anordnung ist in Fig. 1 skizziert (siehe auch Ausführungsbeispiel). Im ersten Teil des Wasserbehandlungssystems liegen bis zum Ausgang vom Reaktor R1 (der zur Sauerstoffentfernung dient) mehr oder weniger ausgeprägte aerobe Bedingungen vor. Dieser Anlagenbereich wird ab dem Ort des Wasserstoffeintrags (Energiequelle) besiedelt, ohne daß Nitrat dabei reduziert wird, da Sauerstoff die Induktion der Nitratreduktase unterdrückt. Wird eines der zuvor genannten anorganischen wasserlöslichen Desinfektionsmittel, insbesondere Natriumhypochlorit zudosiert, bleiben der Wasserstoff-Sättiger die Verbindungsleitungen und der Reaktor R1 etwa bis zur Hälfte des Festbettes in Fließrichtung bewuchsfrei. Der untere Bereich des Festbettes vom Reaktor R1 kann bewachsen, da dort das anorganische Desinfektionsmittel (bspw. das Hypochlorit) neben Sauerstoff katalytisch reduziert ist. In diesem Besiedlungsbereich vollzieht sich in Auslaufrichtung ein Wechsel der Milienbedingungen von aerob über mikroaerophil nach anoxisch. Unter den anoxischen Verhältnissen im Reaktor R2 liegen dann die Voraussetzungen zur Induktion der Nitratreduktase vor; Nitrat wird zu Nitrit umgesetzt. Diese biologische Aktivität überlagert die gegebenenfalls ebenfalls vorhandene chemische Katalyse (falls metalldotierte und/oder -imprägnierte Träger eingesetzt werden, z.B. Pd/Cu-Katalysatoren). Der biologische Nitratumsatz ist unter sonst ungestörten Randbedingungen proportional zur Bewuchsdichte.

Die Bewuchsdichte im Reaktor R2 kann über verschiedene Verfahrensparameter gesteuert werden. Beispielsweise kann der Reaktor R2 als rückspülbarer Festbettreaktor ausgeführt sein und die Biomasse demgemäß durch gelegentliches Rückspülen des Reaktors R2 auf den gewünschten Gehalt begrenzt bzw. eingestellt werden. Weiterhin kann die Biomasse im Reaktor R2 durch Kohlenstofflimitierung, insbesondere über Begrenzung/-Steuerung des Kohlendioxid-Eintrags kontrolliert werden. Eine Variante der Erfindung sieht daher vor, durch Zudosierung von Kohlendioxid (das von den Mikroorganismen als C-Quelle genutzt wird) die Biomasse auf dem Träger in einem stationären Zustand einzupegeln. In anderen Ausgestaltungen der Erfindung kann der Reaktor R2 auch als Wirbelbett oder expandierbares Bett betrieben werden.

Wesentliche Vorteile des erfindungsgemäßen Verfahrens bestehen darin, daß sich unter den erfindungsgemäßen Bedingungen auf dem Träger im Reaktor R2 eine stabile mikrobiologische Mischkultur entwickelt; insbesondere auf solchen Trägern, die zusätzlich mit einem katalytischen Metall, z.B. Pd/Cu, imprägniert sind. Die Mischkultur ist auf den Trägern selbstanhaftend und kann unter den Verfahrensbedingungen mengenmäßig beschränkt werden, so daß keine unerwünschte Überschußbiomasse gebildet wird. Dieses läßt sich insbesondere über C-Limitierung (Begrenzung des Kohlendioxideintrags) erreichen. Der Nitratumsatz, der mit der mikrobiologischen Belegung im Reaktor R2 erzielt werden kann, beträgt bis zum 10-fachen einer an sich üblichen, rein chemischen Katalyse, beispielsweise auf Pd/Cu-Metallbasis als katalytisch aktive Komponente. Die Nitrataktivität isolierter Biologie erreicht Werte größer 300 mg Nitrat/g Trockensubstanz * h; dieses entspricht in etwa einem Faktor von 300 im Vergleich zu einem Katalysator, der noch unbesiedelt rein chemisch-katalytisch (z.B. beim Einfahren einer mit Katalysator im Reaktor R2 bestückten Anlage) arbeitet. Die Aktivität der Biologie in situ (ermittelt aus dem Biotrockensubstanz-Gehalt des besiedelten Trägers/Katalysators und dem Nitratumsatz der kontinuierlichen Anlage) beträgt im Mittel ca. 1500 mg NO₃⁻/g TS * h. Die Ammoniumbildung im eingefahrenen, besiedelten System liegt nach dem Reaktor R2 deutlich unterhalb eines Grenzwertes von 0,5 mg/l. Die Aufwuchssituation auf dem Träger oder einem Katalysator ist sowohl in Betriebsanlagen als auch in Laboranlagen weitgehend identisch; es bilden sich zunächst Biokissen, dann Flocken mit faseriger Struktur aus, bei denen die einzelnen Bakterienzellen in ein Biopolymer, eingebunden sind und dadurch am Träger bzw. Katalysator im Reaktor R2 gut anhaften. Für die Ausbildung dieses polymergebundenen, anhaftenden Zelltypus ist der Selektionsdruck durch das gewählte oxidierende Desinfektionsmittel entscheidend. Nur Spezies, deren Zellen durch Polymerausbildung vor dem toxischen Angriff des Desinfektionsmittels geschützt sind, überleben und besiedeln den Träger oder Katalysator. Die durch das verwendete Desinfektionsmittel im Wasser geschaffene Aktivchlorsituation kann gegebenenfalls auch bereits im eingesetzten Wasser, z.B. in gechlortem Stadtwasser, gegeben sein. Je nach Aktivchlorgehalt kann daher bereits ein ausreichender Desinfektionsmittelgehalt im eingesetzten Wasser vorliegen oder er muß nur noch minimal beispielsweise durch Zugabe von z.B. Hypochlorit ergänzt werden. Ein weiterer Vorteil der erfindungsgemäßen selektierten mikrobiologischen Flora im Reaktor R2 besteht darin, daß sie auch längere Betriebsstillstände ohne Schädigung verträgt und auch Spülvorgänge ohne weiteres möglich sind. Ferner ist auch ein Biologietransfer von Anlage zu Anlage möglich, wodurch ein effektives Animpfen neu anzufahrender Anlagen gewährleistet werden kann.

Die erfindungsgemäß selektierte mikrobiologische Flora hat aufgrund der Polymerbildung der Zellen und aufgrund des dadurch bedingten Anhaftens an den Trägerpartikeln erhebliche Vorteile. So bleibt die Biologie im System, ein ungewolltes Auswaschen wird verhindert. Das System läßt sich stabil auf einem gewünschten Biomasse-Niveau halten, wobei nur ein minimaler Zuwachs im eingefahrenen Reaktionssystem erforderlich ist, um die Abbauaktivität zu erhalten. Die Kontrolle des Bewuchsgrades läßt sich durch gezielte Maßnahmen (beispielsweise C-Limitierung) bewirken. Es tritt keine Verstopfung von nachgeschalteten Filtern auf. Andererseits läßt sich die mikrobiologische Flora aufgrund der Einbettung der Zellen in selbstgebildete Polymermasse und der damit verbundenen speziellen Ausbildung der Biomasse gewünschtenfalls durch geeignete hydrodynamische Maßnahmen gezielt auswaschen. Auch hierdurch wird der Erhalt des gewünschten Grades an Besiedelung bei Spülvorgängen oder der Transfer (für Beimpfungen anderer Reaktoren) ermöglicht.

Die Erfindung betrifft demgemäß auch ein Verfahren zur Aktivierung und/oder Selektion von hochleistungsfähigen Nitrat- und/oder Nitrit-abbauenden Mikroorganismen, das dadurch gekennzeichnet ist, daß man
- einem Nitrat- und/oder Nitrit-belasteten Wasser ein oxidierend wirkendes anorganisches Desinfektionsmittel und Wasserstoffgas zudosiert und unter, gegebenenfalls katalytisch erzeugten, anoxischen Bedingungen
- danach das mit dem Desinfektionsmittel behandelte und mit Wasserstoff beladene Wasser mit einem zur Ansiedlung der zu aktivierenden und/oder zu selektierenden Mikroorganismen dienenden, ggf. metalldotierten und/oder -imprägnierten, gegebenenfalls porösen anorganischen oder organischen Träger kontaktiert,
- und man zumindest bis zum Aufbau einer ausreichenden Nitrat- und/oder Nitrit-abbauenden Biomasse das Verfahren fortführt.

Beim vorstehend beschriebenen Aktivierungs- bzw. Selektions-Verfahren liegen anaerobe bzw. anoxische Bedingungen vor, die hier fakultativ anaerobe Mikroorganismen bevorzugen. Das zu behandelnde Wasser enthält naturgemäß immer eine gewisse Anzahl an vermehrungsfähigen Mikroorganismen, von denen unter den gegebenen Bedingungen (Desinfektionsmitteleinsatz) jedoch nur entsprechend widerstands- und anpassungsfähige, Nitrat- und/oder Nitrit-abbauende Mikroorganismen aktiviert bzw. selektiert werden. Bevorzugte Ausgestaltungen der Aktivierung bzw. Selektion richten sich nach den Bedingungen, wie sie für das erfindungsgemäße Verfahren zum Nitrat- und/oder Nitrit-Abbau oben bereits näher beschrieben sind. Die auf dem Träger angesiedelten aktivierten und/oder selektierten Mikroorganismen können nach an sich üblichen mikrobiologischen Verfahren isoliert werden. Die im erfindungsgemäßen Verfahren aktivierten bzw. selektierten Mikroorganismen sind vorzugsweise Pseudomonas-Spezies, die unter den gegebenen anoxischen und insbesondere nährstoffarmen Bedingungen noch ausreichende Lebens- und Vermehrungsbedingungen vorfinden.

Die Erfindung soll im nachfolgenden anhand weiterer Beispiele erläutert werden, ohne jedoch den Umfang hierdurch zu beschränken.

### Beispiele

Die Erfindung wird nachfolgend an einer beispielhaften, kontinuierlich arbeitenden Anlage verdeutlicht, die aus einer Kaskaden-Anordnung von 3 oder weiteren Reaktoren sowie weiteren üblichen Anlagenteilen bestand. Die Anlage umfaßte als weitere übliche Anlagenteile insbesondere eine den Wasserdurchfluß regulierende Pumpe mit variabler Förderleistung, Begasungseinrichtungen (Sättiger), in denen das Wasser mit Wasserstoffgas gegebenenfalls unter Druck begast und innig vermischt wurde, Rohrleitungen und Meß- und Kontrolleinrichtungen zur Bestimmung von pH, Temperatur, Druck und gewünschtenfalls weiteren Parametern. Die Anordnung der jeweiligen Anlagenteile und die Verfahrensführung ist schematisch in Figur 1 erläutert.

Dem zu behandelnden Wasser wurde zunächst Natriumhypochlorit (in Wasser gelöst) in einer Menge zudosiert, so daß sich im zu behandelnden Wasser eine Konzentration von 0,1 bis 0,3 mg/l einstellte (berechnet als freies Aktivchlor); gewünschtenfalls wurde zur Steuerung des mikrobiologischen Wachstums im Reaktor R2 zusätzlich Kohlendioxid zugefahren. Das mit dem Desinfektionsmittel behandelte Wasser wurde über einen Wasserstoffsättiger geführt und danach in den Reaktor R1 eingeleitet. Im Reaktor R1 wurden an einem Palladium-Katalysator (Palladium auf anorganischem Träger) anoxische Bedingungen durch Entfernung von Sauerstoff aus dem zu behandelnden Wasser eingestellt; im Reaktor R1 herrscht Propfenströmung. Es wurde kein Wachstum von Mikroorganismen beobachtet, bis das zugesetzte Natriumhypochlorit reduktiv umgesetzt war. Im Reaktor R2 fand dann die Ansiedlung der mikrobiologischen Mischkultur statt, entsprechend der zuvor erfolgten Selektion unter Hypochlorit und anoxischen Bedingungen. In der Mischkultur wurde als Haupt-Spezies Pseudomonas pseudoflava (Hydrogenophaga pseudoflava) bestimmt. Eventuell hygienisch bedenkliche Keime waren nicht vorhanden. Das Wasser wurde dann über einen zweiten Wasserstoffsättiger in einen Reaktor R3 überführt.

Der Träger im Reaktor R2 (für die Ansiedlung der Mikrobiologie) war im vorliegenden Fall ein zur Nitrat/Nitrit-Reduktion selbst befähigter Katalysator mit Pd/Cu als katalytisch aktive Komponente auf einen Träger, wie dieser in der nachfolgenden Tabelle I spezifiziert wird (siehe (B)b)). Der Reaktor R2 wurde als expandiertes Bett betrieben. Der Reaktor R3 enthielt einen Palladium-Trägerkatalysator in Form eines expandierten Bettes. Wahlweise kann ein weiterer Reaktor R4 in gleicher Ausführung zum Reaktor R3 nachgeschaltet werden.

**Tabelle I:**

| Träger und Katalysatoren, insbesondere auch zur Besiedelung mit selektierten Nitrat- und/oder Nitrit-abbauenden Mikroorganismen | | |
|---|---|---|
| | Träger/Katalysator 1 | Träger/Katalysator 2 |
| (A) Träger: | | |
| Material | Al₂O₃ | Al₂O₃ |
| | | |
| Herstellung | Granulation (z.B. WO 93/17790) | Sprühgelierung (z.B. WO 94/20202 bzw. WO 94/20203) |
| | | |
| Partikelgröße | 400 - 600 µm | 400 - 600 µm |
| | | |
| Schüttdichte | 0,9 g/ml | 0,6 g/ml |
| | | |
| spezifische BET-Oberfläche | ca. 40 m²/g | ca. 145 m²/g |
| | | |
| Porenvolumen | 0,4 ml/g | 0,6-1,0 ml/g |
| | | |
| häufigster Porendurchmesser (HPD) | ca. 75 nm | ca. 18 nm |

| (B) Katalysator | | |
|---|---|---|
| Katalytische | a) 2 % Pd | a) 1 % Pd |
| Komponente | b) 2 % Pd und | b) 1 % Pd und |
| auf Träger wie oben angegeben | 0,5 % Cu | 0,25 % Cu |

Die in der Tabelle I beschriebenen Katalysatorträger (A) eigneten sich nicht nur zur Ansiedelung der mikrobiologischen Flora im Reaktor R2, sondern wurden nach entsprechender Belegung mit katalytischer Komponente als Katalysatoren (B) sowohl zur Sauerstoffentfernung im Reaktor R1 (Palladium-Belegung) als auch zum ergänzenden Nitrat- und/oder Nitrit-Abbau im Reaktor R3 bzw. im weiteren Reaktor R4 eingesetzt. Wurde im Reaktor R2 bereits ein Katalysator, der zum Nitrat- und/-oder Nitrit-Abbau geeignet ist, eingesetzt, so konnte das Verfahren bereits von Beginn an, d.h. bereits während der Kolonisierungsphase im Reaktor R2, zur Wasserbehandlung eingesetzt werden. Nachdem eine ausreichende mikrobiologische Flora auf dem Träger bzw. dem Katalysator in Reaktor R2 aufgewachsen war, wurde das Verfahren in kontinuierlicher Weise fortgeführt, wobei die aufgewachsene mikrobiologische Flora zu einer unerwartet hohen Intensivierung des Nitrat- und/oder Nitritabbaues beitrug. Hierbei bewirkte die mikrobiologische Flora in etwa den 5-fachen Umsatz des reinen Palladium/Kupfer-Katalysators. Die Nitrataktivität isolierter Biologie pro Stunde erreichte hierbei Werte von größer 300 mg Nitrat pro g Trockensubstanz; dies entspricht in etwa einem Faktor von 300 im Vergleich zum Katalysator, der noch unbesiedelt rein chemisch-katalytisch (beim Einfahren der mit Katalysator im Reaktor R2 bestückten Anlage) arbeitet. Die Aktivität der Biologie in situ (ermittelt aus dem Bio-Trockensubstanz-Gehalt des besiedelten Katalysators und dem Nitratumsatz der kontinuierlichen Anlage) betrug im Mittel somit ca. 1500 mg NO₃⁻/g TS * h.

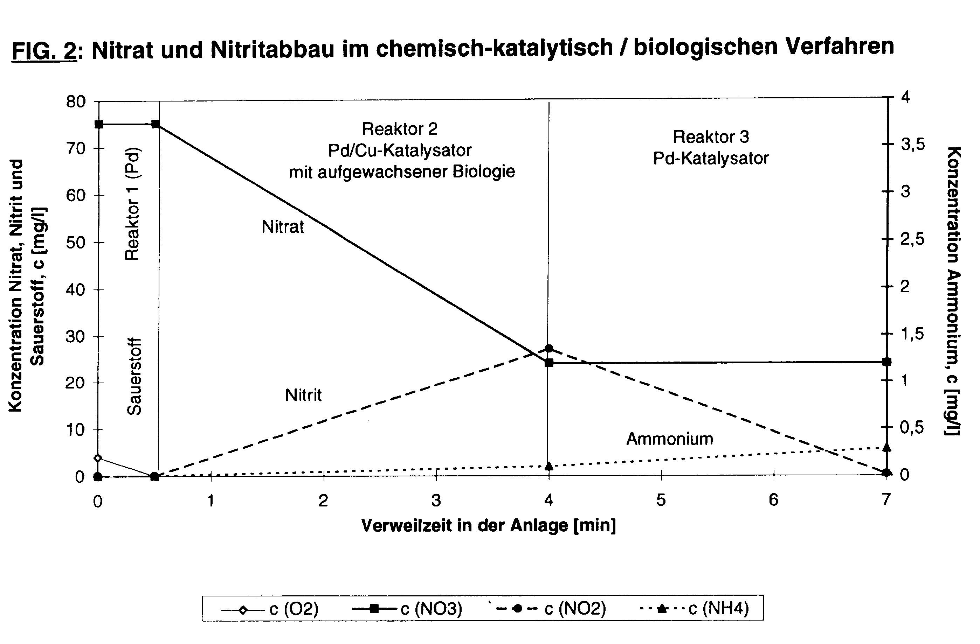

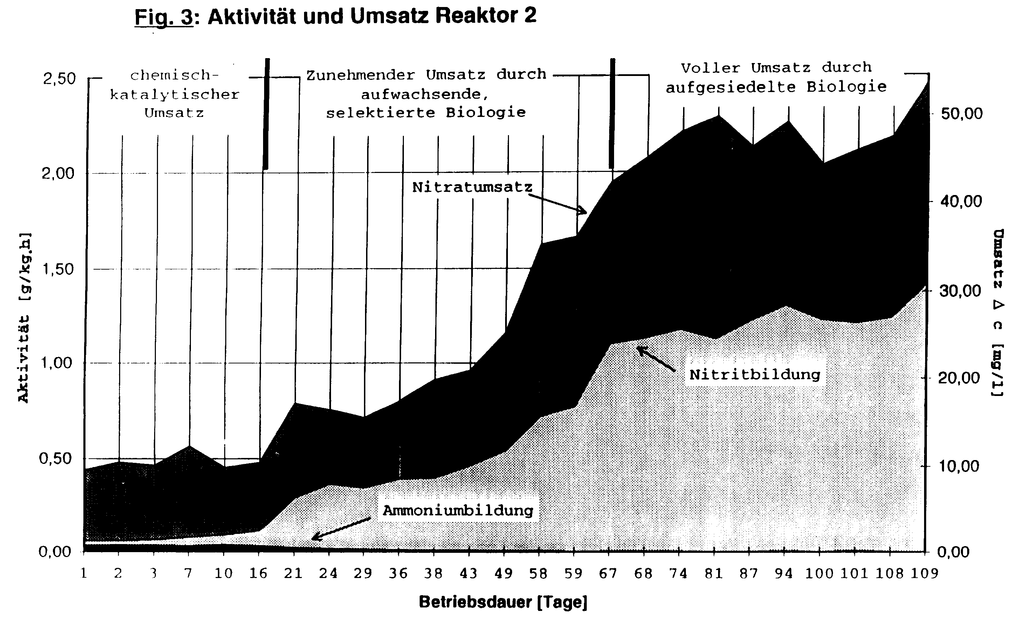

Die Nitrat- und/oder Nitrit-Abbauleistung der Anlage ist in Figur 2 verdeutlicht. Die Leistung der mikrobiologischen Flora im Reaktor R2 ist weiterhin in der Figur 3 erläutert. Bei der aufgewachsenen mikrobiologischen Flora handelt es sich insbesondere um eine solche, die verstärkt zunächst Nitrat zu Nitrit umsetzt, welches dann im nachgeschalteten Reaktor R3 effizient zu Stickstoff abgebaut werden kann.

Das beschriebene Verfahren zeigt sehr deutlich die Vorteile der Kombination des chemisch-katalytischen Abbaues von Nitrat und Nitrit in Verbindung mit hoher biologischer Aktivität.

## Patentansprüche

1. Verfahren zur Entfernung oder Verminderung des Nitrat- und/oder Nitrit-Gehaltes in Nitrat- und/oder Nitrit-belastetem Wasser unter selektiver Stickstoffbildung, dadurch gekennzeichnet, daß man das Nitrat- und/oder Nitrit-belastete Wasser unter Zudosierung von Wasserstoffgas und von wasserlöslichem anorganischem Desinfektionsmittel unter anoxischen Bedingungen mit einer auf einem anorganischen oder organischen Träger angesiedelten, hochleistungsfähigen Nitrat- und/oder Nitrit-abbauenden mikrobiologischen Flora kontaktiert, wobei diese mikrobiologische Flora aus natürlicherweise im zu behandelnden Wasser vorhandenen Mikroorganismen vor und/oder während des Verfahrens unter dem Selektionsdruck des zudosierten wasserlöslichen anorganischen Desinfektionsmittels und den anoxischen Bedingungen auf dem Träger selbst anhaftend kolonisiert und dabei an die Verfahrensbedingungen zur Entfernung oder Verminderung des Nitrat- und/oder Nitrit-Gehaltes aus Wasser adaptiert wird.

2. Verfahren nach Anspruch 1 zur Entfernung oder Verminderung des Nitrat- und/oder Nitrit-Gehaltes in Nitrat- und/-oder Nitrit-belastetem Wasser unter selektiver Stickstoffbildung, dadurch gekennzeichnet, daß man das Nitrat- und/oder Nitrit-belastete Wasser unter Zudosierung von Wasserstoffgas und eines anorganischen wasserlöslichen Desinfektionsmittels
a) zur Herstellung von anoxischen Bedingungen zunächst über einen mit Katalysator befüllten Reaktor R1 führt, in dem im Wasser enthaltener Sauerstoff mit Wasserstoff katalytisch reduziert wird,
b) das Wasser über einen zweiten, mit einem anorganischen oder organischen Träger befüllten Reaktor R2 führt, der zur Ansiedlung einer hochleistungsfähigen Nitrat- und/oder Nitrit-abbauenden mikrobiologischen Flora dient, wobei diese mikrobiologische Flora aus natürlicherweise im zu behandelnden Wasser vorhandenen Mikroorganismen unter dem Selektionsdruck des zudosierten wasserlöslichen anorganischen Desinfektionsmittels und den anoxischen Bedingungen auf dem Träger selbstanhaftend kolonisiert und an die Verfahrensbedingungen zur Entfernung oder Verminderung des Nitrat- und/oder Nitrit-Gehaltes aus dem Wasser adaptiert wird,
c) und man danach das Wasser, gegegebenfalls unter Zudosierung von weiterem Desinfektionsmittel und/oder Wasserstoffgas über zumindest einen weiteren mit Katalysator zur Nitrat- und/oder Nitrit-Entfernung gefüllten Reaktor R3 führt und danach das Wasser aus dem Verfahren entläßt.

3. Verfahren nach Anspruch 1 oder 2, dadurch gekennzeichnet, daß als Desinfektionsmittel ein oxidierend wirkendes anorganisches Mittel eingesetzt wird, vorzugsweise Hypochlorit in der Salzform, insbesondere in Form des Natriumsalzes, oder Chlor oder Chlordioxid.

4. Verfahren nach Anspruch 3, dadurch gekennzeichnet, daß als Desinfektionsmittel Natriumhypochlorit eingesetzt wird.

5. Verfahren nach Anspruch 3 oder 4, dadurch gekennzeichnet, daß das Desinfektionsmittel in einer Menge von 0,05 bis 0,5 mg /1 Wasser (berechnet als freies Aktivchlor), vorzugsweise von 0,1 bis 0,3 mg/l, zudosiert wird.

6. Verfahren nach Anspruch 1 oder 2, dadurch gekennzeichnet, daß die mikrobiologische Flora auf einem anorganischen Träger angesiedelt ist, welcher im wesentlichen aus Aluminiumoxid (Al₂O₃) besteht und durch folgende Eigenschaften gekennzeichnet ist:
aI) er besteht aus Aluminiumoxid der Modifikation "Gamma" und "Kappa", wobei die Modifikation "Delta" in geringer Menge enthalten sein kann, oder
aII) er besteht aus Aluminiumoxid der Modifikation "Theta" und "Kappa", wobei die Modifikation "Alpha" in geringer Menge enthalten sein kann;
b) die Verteilung der Porendurchmesser weist ein Maximum (Monomodale Porenverteilung) oder zwei Maxima (bimodale Porenverteilung) im Bereich zwischen 100 und 1500 Å auf;
c) die BET-Oberfläche liegt zwischen 20 und 200 m²/g.

7. Verfahren nach Anspruch 6, dadurch gekennzeichnet, daß der im wesentlichen aus Aluminiumoxid bestehende Träger entweder ein Maximum (monomodale Porenverteilung) in der Porenverteilung im Bereich von 500 bis 1500 Å aufweist oder zwei Maxima (bimodale Porenverteilung) in der Porenverteilung im Bereich von 100 bis 1500 Å aufweist.

8. Verfahren nach Anspruch 1 oder 2, dadurch gekennzeichnet, daß ein anorganischer Träger auf Basis von Aluminiumoxid - gegebenenfalls mit in das Aluminiumoxid eingebrachten Anteilen anorganischer Oxide aus der Gruppe Magnesiumoxid, Siliciumdioxid, Alumosilikat, Titandioxid, Manganoxid, Eisenoxid, Molybdänoxid, Vanadiumoxid, und/oder Zirkoniumoxid eingesetzt wird, wobei der Träger entweder durch Granulation oder durch Überführen eines Sols in kugelförmige Solteilchen unter Kontaktieren der Solteilchen mit einem Reaktionsgas und Auffangen der Solteilchen in einer Auffangvorrichtung, Aufarbeiten der verfestigten Solteilchen durch Altern, Waschen, Trocknen und Kalzinieren, hergestellt wird, mit der Maßgabe, daß der Träger eine spezifische BET-Oberfläche im Bereich von 5 bis 250 m²/g, vorzugsweise im Bereich 100 bis 200 m²/g, aufweist.

9. Verfahren nach einem der vorhergehenden Ansprüche 6 bis 8, dadurch gekennzeichnet, daß man einen mit einem katalytisch wirkenden Übergangsmetall imprägnierten porösen Träger im Reaktor R2 einsetzt, vorzugsweise einen mit einem oder mehreren Metallen aus der Gruppe Palladium, Rhodium und Kupfer imprägnierten Träger.

10. Verfahren nach Anspruch 9, dadurch gekennzeichnet, daß man einen mit Palladium und/oder Kupfer imprägnierten anorganischen porösen Träger im Reaktor R2 einsetzt.

11. Verfahren nach einem der vorhergehenden Ansprüche, dadurch gekennzeichnet, daß an einer oder mehreren Stellen des Verfahrens Kohlendioxid zudosiert wird.

12. Verfahren zur Aktivierung und/oder Selektion von hochleistungsfähigen Nitrat- und/oder Nitrit-abbauenden Mikroorganismen, dadurch gekennzeichnet, daß man
- einem Nitrat- und/oder Nitrit-belasteten Wasser ein oxidierend wirkendes anorganisches Desinfektionsmittel und Wasserstoffgas zudosiert und unter, gegebenenfalls katalytisch erzeugten, anoxischen Bedingungen
- danach das mit dem Desinfektionsmittel behandelte und mit Wasserstoff beladene Wasser mit einem zur Ansiedlung der zu aktivierenden und/oder zu selektierenden Mikroorganismen dienenden, ggf. metalldotierten und/oder -imprägnierten, gegebenenfalls porösen anorganischen oder organischen Träger kontaktiert,
- und man zumindest bis zum Aufbau einer ausreichenden Nitrat- und/oder Nitrit-abbauenden Biomasse das Verfahren fortführt.

## Claims

1. Process for removing or decreasing the nitrate and/or nitrite content in nitrate- and/or nitrite-polluted water with selective nitrogen formation, characterized in that nitrate- and/or nitrite-polluted water, with addition of hydrogen gas and water-soluble inorganic disinfectant, is contacted under anoxic conditions with a high-efficiency nitrate- and/or nitrite-degrading microbiological flora established on an inorganic or organic support, this microbiological flora of microorganisms naturally present in the water to be treated colonizing the support itself by attachment before and/or during the process under the selection pressure of the added water-soluble inorganic disinfectant and the anoxic conditions and thus becoming adapted to the process conditions for removing or decreasing the nitrate and/or nitrite content of the water.

2. Process according to Claim 1 for removing or decreasing the nitrate and/or nitrite content in nitrate-and/or nitrite-polluted water with selective nitrogen formation, characterized in that the nitrate- and/or nitrite-polluted water, with addition of hydrogen gas and an inorganic water-soluble disinfectant,
a) is first, to produce anoxic conditions, conducted via a catalyst-filled reactor R1 in which oxygen contained in the water is catalytically reduced with hydrogen,
b) the water is conducted via a second reactor R2 filled with an inorganic or organic support which serves to establish a high-efficiency nitrate-and/or nitrite-degrading microbiological flora, this microbiological flora of microorganisms naturally present in the water to be treated colonizing the support itself by attachment under the selection pressure of the added water-soluble inorganic disinfectant and the anoxic conditions and becoming adapted to the process conditions for removing or decreasing the nitrate and/or nitrite content of the water,
c) and the water is then, optionally with addition of further disinfectant and/or hydrogen gas, conducted via at least one further reactor R3 filled with catalyst for the removal of nitrate and/or nitrite and the water is then released from the process.

3. Process according to Claim 1 or 2, characterized in that the disinfectant used is an oxidizing inorganic composition, preferably hypochlorite in the salt form, in particular in the form of the sodium salt, or chlorine or chlorine dioxide.

4. Process according to Claim 3, characterized in that the disinfectant used is sodium hypochlorite.

5. Process according to Claim 3 or 4, characterized in that the disinfectant is added in an amount of 0.05 to 0.5 mg/l of water (calculated as free active chlorine), preferably 0.1 to 0.3 mg/l.

6. Process according to Claim 1 or 2, characterized in that the microbiological flora colonizes an inorganic support which essentially comprises alumina (Al₂O₃) and is characterized by the following properties:
aI) it comprises alumina of the modification "gamma" and "kappa", the modification "delta" being able to be present in a small amount, or
aII) it comprises alumina of the modification "theta" and "kappa", the modification "alpha" being able to be present in a small amount;
b) the distribution of the pore diameters has one maximum (monomodal pore distribution) or two maxima (bimodal pore distribution) in the range between 100 and 1500 Å;
c) the BET surface area is between 20 and 200 m²/g.

7. Process according to Claim 6, characterized in that the support, essentially comprising alumina, has either one maximum (monomodal pore distribution) in the pore distribution in the range from 500 to 1500 Å or has two maxima (bimodal pore distribution) in the pore distribution in the range from 100 to 1500 Å.

8. Process according to Claim 1 or 2, characterized in that an inorganic support based on alumina is used - optionally having incorporated into the alumina portions of inorganic oxides selected from the group consisting of magnesium oxide, silicon dioxide, aluminosilicate, titanium dioxide, manganese oxide, iron oxide, molybdenum oxide, vanadium oxide and/or zirconium oxide, the support being produced either by granulation or by conversion of a sol into spherical sol particles with contacting of the sol particles with a reaction gas and collecting the sol particles in a collection apparatus, and workup of the solidified sol particles by ageing, washing, drying and calcination, with the proviso that the support has a specific BET surface area in the range from 5 to 250 m²/g, preferably in the range from 100 to 200 m²/g.

9. Process according to one of the preceding Claims 6 to 8, characterized in that a porous support impregnated with a catalytically active transition metal is used in the reactor R2, preferably a support impregnated with one or more metals selected from the group consisting of palladium, rhodium and copper.

10. Process according to Claim 9, characterized in that an inorganic porous support impregnated with palladium and/or copper is used in the reactor R2.

11. Process according to one of the preceding claims, characterized in that carbon dioxide is added at one or more points of the process.

12. Process for the activation and/or selection of high-efficiency nitrate- and/or nitrite-degrading microorganisms, characterized in that
- an oxidizing inorganic disinfectant and hydrogen gas are added to a nitrate- and/or nitrite-polluted water and under anoxic conditions, optionally generated catalytically
- the disinfectant-treated and hydrogen-laden water is then contacted with an, optionally metal-doped and/or metal-impregnated, optionally porous, inorganic or organic support serving for colonization by the microorganisms to be activated and/or selected,
- and the process is continued at least until a sufficient nitrate- and/or nitrite-degrading biomass is built up.

## Revendications

1. Procédé d'élimination ou de diminution de la teneur en nitrates et/ou en nitrites dans une eau polluée par des nitrates et/ou par des nitrites par formation sélective d'azote, caractérisé en ce que l'on met en contact l'eau polluée par des nitrates et/ou par des nitrites, avec addition d'hydrogène gazeux et d'un agent désinfectant inorganique soluble dans l'eau en conditions anoxiques, avec une flore microbiologique à rendement élevé, décomposant les nitrates et/ou les nitrites, implantée sur un support inorganique ou organique, cette flore microbiologique provenant de micro-organismes naturellement présents dans l'eau à traiter, colonisant en y adhérant le support lui-même avant et/ou au cours du procédé sous la pression de sélection exercée par l'agent désinfectant ajouté inorganique soluble dans l'eau et par les conditions anoxiques, et étant ainsi adaptée aux conditions du procédé pour l'élimination ou la diminution de la teneur en nitrates et/ou en nitrites de l'eau.

2. Procédé suivant la revendication 1, pour l'élimination ou la diminution de la teneur en nitrates et/ou en nitrites dans une eau polluée par des nitrates et/ou par des nitrites par formation sélective d'azote, caractérisé en ce que l'eau polluée par des nitrates et/ou des nitrites, après addition d'hydrogène gazeux et d'un agent désinfectant inorganique soluble dans l'eau
(a) est d'abord amenée, pour l'établissement de conditions anoxiques, dans un réacteur R1 rempli de catalyseur, dans lequel l'oxygène contenu dans l'eau est réduit catalytiquement avec de l'hydrogène,
(b) est amenée dans un deuxième réacteur R2, rempli d'un support inorganique ou organique, qui sert à l'implantation d'une flore microbiologique à rendement élevé, décomposant les nitrates et/ou les nitrites, cette flore microbiologique, provenant de micro-organismes naturellement présents dans l'eau à traiter, colonisant en y adhérant le support lui-même sous la pression de sélection exercée par l'agent désinfectant ajouté inorganique soluble dans l'eau et par les conditions anoxiques, et étant ainsi adaptée aux conditions du procédé pour l'élimination ou la diminution de la teneur en nitrates et/ou en nitrites de l'eau, et
(c) est ensuite amenée, éventuellement avec addition d'agent désinfectant et/ou d'hydrogène gazeux supplémentaires, dans au moins un autre réacteur R3 rempli de catalyseur pour l'élimination des nitrates et/ou des nitrites, après quoi l'eau est évacuée du procédé.

3. Procédé suivant la revendication 1 ou 2, caractérisé en ce que l'agent désinfectant utilisé est un agent inorganique agissant par oxydation, de préférence un hypochlorite sous forme de sel, en particulier sous la forme du sel de sodium, ou le chlore ou le dioxyde de chlore.

4. Procédé suivant la revendication 3, caractérisé en ce que l'agent de désinfection utilisé est l'hypochlorite de sodium.

5. Procédé suivant la revendication 3 ou 4, caractérisé en ce que l'agent de désinfection est ajouté en une quantité de 0,05 à 0,5 mg/l d'eau (exprimée en chlore actif libre), de préférence de 0,1 à 0,3 mg/l.

6. Procédé suivant la revendication 1 ou 2, caractérisé en ce que la flore microbiologique est implantée sur un support inorganique qui est essentiellement composé d'oxyde d'aluminium (Al₂O₃) et qui est caractérisé par les propriétés suivantes :
aI) il est composé d'oxyde d'aluminium sous forme "Gamma" et "Kappa", la forme "Delta" pouvant être contenue en quantité moindre, ou
aII) il est composé d'oxyde d'aluminium sous forme "Thêta" et "Kappa", la forme "Alpha" pouvant être contenue en quantité moindre;
b) la distribution des diamètres des pores présente un maximum (distribution monomodale des pores) ou deux maxima (distribution bimodale des pores) dans l'intervalle compris entre 100 et 1500 Å, et
c) la surface du "BET" se situe entre 20 et 200 m²/g.

7. Procédé suivant la revendication 6, caractérisé en ce que le support essentiellement composé d'oxyde d'aluminium présente soit un maximum (distribution monomodale des pores) dans la distribution des pores, qui se situe dans l'intervalle de 500 à 1500 Å, soit deux maxima (distribution bimodale des pores) dans la distribution des pores, qui se situent dans l'intervalle de 100 à 1500 Å.

8. Procédé suivant la revendication 1 ou 2, caractérisé en ce que l'on utilise un support inorganique à base d'oxyde d'aluminium, éventuellement avec des fractions, apportées avec l'oxyde d'aluminium, du groupe de l'oxyde de magnésium, du dioxyde de silicium, du silicate d'aluminium, du dioxyde de titane, de l'oxyde de manganèse, de l'oxyde de fer, de l'oxyde de molybdène, de l'oxyde de vanadium et/ou de l'oxyde de zirconium, le support étant préparé soit par granulation soit par transformation d'un sol en particules de sol sphériques, par contact des particules du sol avec un gaz réactif et captage des particules du sol dans un dispositif de captage, traitement des particules du sol solidifiées par vieillissement, lavage, séchage et calcination, avec la condition que le support présente une surface "BET" spécifique comprise dans l'intervalle de 5 à 250 m²/g, de préférence dans l'intervalle de 100 à 200 m²/g.

9. Procédé suivant l'une quelconque des revendications précédentes 6 à 8, caractérisé en ce que l'on utilise dans le réacteur R2 un support poreux imprégné d'un métal de transition à action catalytique, de préférence un support imprégné d'un ou plusieurs métaux du groupe du palladium, du rhodium et du cuivre.

10. Procédé suivant la revendication 9, caractérisé en ce que l'on utilise dans le réacteur R2 un support poreux inorganique imprégné de palladium et/ou de cuivre.

11. Procédé suivant l'une quelconque des revendications précédentes, caractérisé en ce que l'on ajoute du dioxyde de carbone à une ou plusieurs étapes du procédé.

12. Procédé d'activation et/ou de sélection de micro-organismes à rendement élevé décomposant les nitrates et/ou les nitrites, caractérisé en ce que
- l'on ajoute à une eau polluée par des nitrates et/ou par des nitrites un agent de désinfection inorganique agissant par oxydation et de l'hydrogène gazeux et dans des conditions anoxiques, éventuellement produites catalytiquement;
- l'on met ensuite en contact l'eau traitée par l'agent désinfectant et chargée d'hydrogène avec un support inorganique ou organique, éventuellement dopé et/ou imprégné par un métal, éventuellement poreux, servant à l'implantation des micro-organismes à activer et/ou à sélectionner, et
- l'on poursuit le procédé au moins jusqu'à la formation d'une biomasse suffisante décomposant les nitrates et/ou les nitrites.
